(19) Europäisches Patentamt
European Patent Office
Office européen des brevets

(11) **EP 0 587 312 B1**

(12) **EUROPEAN PATENT SPECIFICATION**

(45) Date of publication and mention
of the grant of the patent:
**23.07.2003 Bulletin 2003/30**

(51) Int Cl.⁷: **C12N 15/10**, C12Q 1/68,
C07K 1/00, C12P 21/08

(21) Application number: **93306259.8**

(22) Date of filing: **09.08.1993**

(54) **Polypeptide production**

Polypeptidproduktion

Production de polypeptides

(84) Designated Contracting States:
**AT BE CH DE DK ES FR GB GR IE IT LI NL PT SE**

(30) Priority: **11.08.1992 GB 9216983**

(43) Date of publication of application:
**16.03.1994 Bulletin 1994/11**

(73) Proprietors:
• **UNILEVER PLC**
**London EC4P 4BQ (GB)**
Designated Contracting States:
**GB IE**
• **UNILEVER N.V.**
**3013 AL Rotterdam (NL)**
Designated Contracting States:
**BE CH DE DK ES FR GR IT LI NL PT SE AT**

(72) Inventor: **Berry, Mark John**
**Wellingborough, Northants NN10 8DQ (GB)**

(56) References cited:
**EP-A- 0 368 684**     **WO-A-93/03151**

• **IMMUNOLOGY LETTERS, vol.33, no.1, June 1990, ELSEVIER, AMSTERDAM, NL; pages 53 - 59 M. SUTER ET AL. 'Rabbit single domain antibodies specific to protein C expressed in procaryotes'**
• **BIO/TECHNOLOGY, vol.9, no.3, March 1991, NATURE AMERICA, INC., NEW YORK, US; pages 273 - 278 A. SKERRA ET AL. 'The functional expression of antibody Fv fragments in Escherichia coli: Improved vectors and a generally applicable purification technique'**
• **TRENDS IN BIOTECHNOLOGY, vol.8, no.4, April 1990, ELSEVIER SCIENCE PUBLISHERS,LTD.,CAMBRIDGE,UK; pages 88 - 93 H.M. SASSENFELD 'Engineering proteins for purification'**
• **NATURE, vol.341, 12 October 1989, MACMILLAN JOURNALS LTD., LONDON, UK; pages 544 - 546 E. SALLY ET AL. 'Binding activities of a repertoire of single immunglobulin variable domains secreted from Escherichia coli'**
• **PROC. NATL. ACAD SCI., vol.86, no.10, May 1989, NATL. ACAD SCI., WASHINGTON, DC, US; pages 3833 - 3837 R. ORLANDI ET AL. 'Cloning immunoglobulin variable domains for expression by the polymerase chain reaction'**
• **J. IMMUNOL. METHODS, vol.167, no.1-2, 3 January 1994, ELSEVIER,AMSTERDAM, NL; pages 173 - 182 M.J. BERRY ET AL. 'Assay and purification of Fv fragments in fermenter cultures: design and evaluation of generic binding reagents'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

This invention is concerned with the production of polypeptides by means of recombinant DNA technology. This is a subject of progressively growing commercial importance. In particular the invention is applicable to the production of polypeptides which are antibody fragments, although it is not confined to these polypeptides.

When a polypeptide is produced by means of recombinant DNA technology the necessary DNA sequence which codes for the desired polypeptide must first be obtained. This extra genetic material is then introduced into an organism (frequently the bacterial species E.coli) and the resulting transformed organism is then cultured so that it produces the desired polypeptide which is harvested from the culture.

When culturing the transformed non-human organism it will often be necessary to assay for the desired polypeptide. For instance, this may be required during development work to optimise the growth conditions of a transformed microorganism when it will be desirable to assess the effect of a change in pH, or temperature or formulation of the growth medium. Later, during production an assay will be desirable to check production performance.

Assay techniques with the required sensitivity and specificity already exist. Two conventional techniques which are suitable are ELISA and Western Blot.

However, for these assay techniques, it is necessary to have available an antibody (or possibly an antibody fragment or other binding reagent) which exhibits specific binding affinity for the desired polypeptide. In the context of these assay techniques such an antibody may be referred to as a "tracer antibody".

Thus, in order to be able to assay for a desired polypeptide it is first necessary to obtain a supply of antibody (or fragments or other binding reagent) with specific binding affinity to the desired polypeptide.

An antibody or antibody fragment or other reagent with specific binding affinity to a desired polypeptide may also be required for purification of the peptide, for example by affinity chromatography. An antibody for this purpose may be referred to as a "capture antibody".

It will thus be appreciated that for every desired polypeptide there is likely to be a requirement for a binding reagent such as an antibody with specific binding affinity to the polypeptide. In many instances this will not be available and its provision will be an extra task in putting a desired polypeptide into production.

This requirement is particularly acute when the desired polypeptide is an antibody fragment which is wholly or largely formed by the variable region of an antibody. By definition these are highly variable and there is no pre-existing source of suitable antibodies.

One approach to this has been to transform an non-human organism with fused genes so that is expresses the desired polypeptide fused onto an additional peptide against which an antibody is available. This additional peptide which may be referred to as a "tag" or "tail" can then be used in purification and/or assay. This is discussed by Sassenfeld in Tibtech 8, 88 (April 1990). Possible disadvantages arising from such tags are recognised.

Ward et al., Nature 341, 544-546, 1989, disclosed a method of using an antibody specific to a peptide tag for detecting expressed VH domains from gene libraries in E. coli. In this method, the VH genes were amplified by PCR and then cloned for expression into a vector which incorporated a c-terminal peptide tag into the cloned amplified DNA. The peptide tag resulting from cloning into the vector was used to facilitate detection of the expressed VH domains with antibodies against the peptide tag.

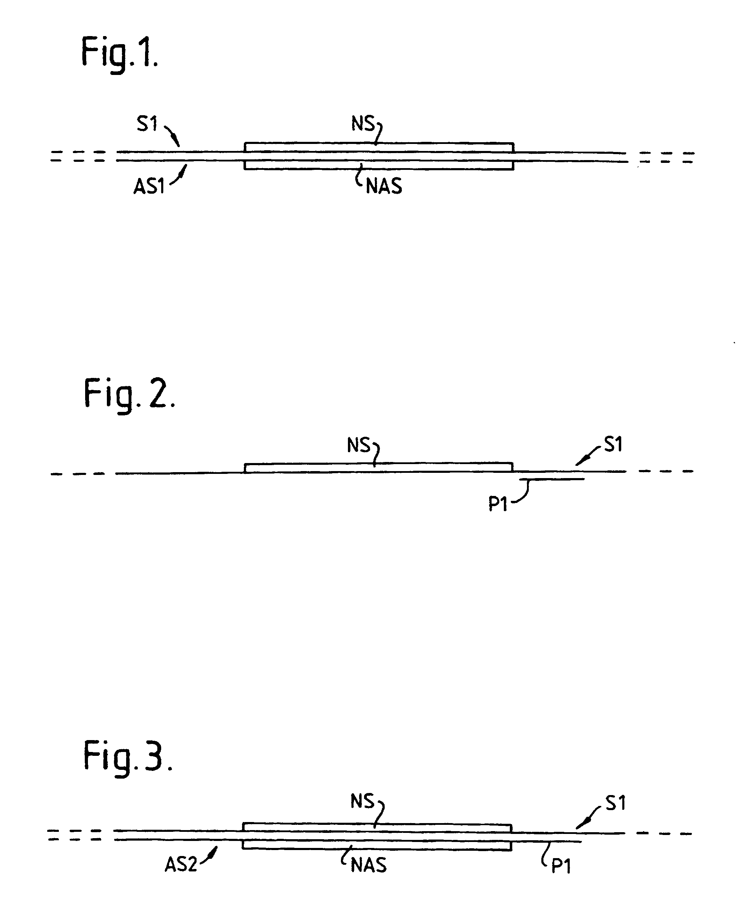

The present inventors have now appreciated that antibodies to desired polypeptides can be obtained by taking advantage of a side effect of a well known technique used in gene cloning, namely the polymerase chain reaction (PCR). This technique "amplifies" the desired nucleotide sequence by preferentially replicating this sequence. The characteristics of the PCR technique will now be pointed out with reference to Figs. 1 to 6 of the accompanying drawings, which are diagrams illustrating DNA replication.

The procedure for gene amplification by the polymerase chain reaction starts with DNA (deoxynucleic acid) which includes a gene (a nucleotide sequence) coding for a desired polypeptide. Other nucleotide sequences are also present. For instance when it is intended to produce a fragment of a monoclonal antibody, the starting point for the polymerase chain reaction may be the genome of the hybridoma cells which produce the monoclonal antibody concerned. More usually, the starting DNA is cDNA artificially obtained by reverse transcription of mRNA recovered from hybridoma cells.

The polymerase chain reaction requires a pair of "primers" each of which is a relatively short nucleotide sequence able to anneal to a portion of the DNA strand adjoining a respective end of the desired gene. One primer is able to anneal with the sense strand at one end of the desired gene, the other with the antisense strand at the opposite end of the desired gene. Each primer used for the polymerase chain reaction may be a single oligonucleotide or may be a mixture of several oligonucleotides which are similar. Such a mixture of similar oligonucleotides may be referred to as a degenerate primer.

The polymerase chain reaction utilises a sequence of steps which are carried out in a repeated cycle. In a particularly convenient form, these steps are controlled by temperature. Initially a reaction mixture is prepared containing the starting genetic material, the two primers, monomeric nucleotides and DNA polymerase.

As illustrated in Fig. 1 the starting genetic material is double-stranded DNA including the desired gene (nucleotide sequence) with other nucleotide sequences at either side of this. S1 denotes the sense strand, with the desired nucleotide sequence designated NS; AS1 denotes the antisense strand with the (complementary) desired sequence NAS. In a first step this double-stranded DNA is denatured to single-stranded DNA by heating, for example to about 95°C for one minute.

Next it is cooled to a temperature at which annealing of DNA occurs, for example 30-65°C for about one minute. However, because this takes place in the presence of primer, some primer can anneal to the DNA adjoining one end of the desired nucleotide sequence. This is illustrated in Fig. 2 where primer P1 has annealed to the sense strand SI of the original DNA. In the third step of the cycle the annealed DNA is incubated at a suitable temperature for the synthesis of new DNA extending from the primer (e.g. 75°C using thermophilic DNA polymerase). This leads to the formation of a new antisense DNA strand of indefinite length but with the primer P1 and the desired nucleotide sequence NAS at one end. This is shown in Fig. 3 where the new antisense strand is designated AS2.

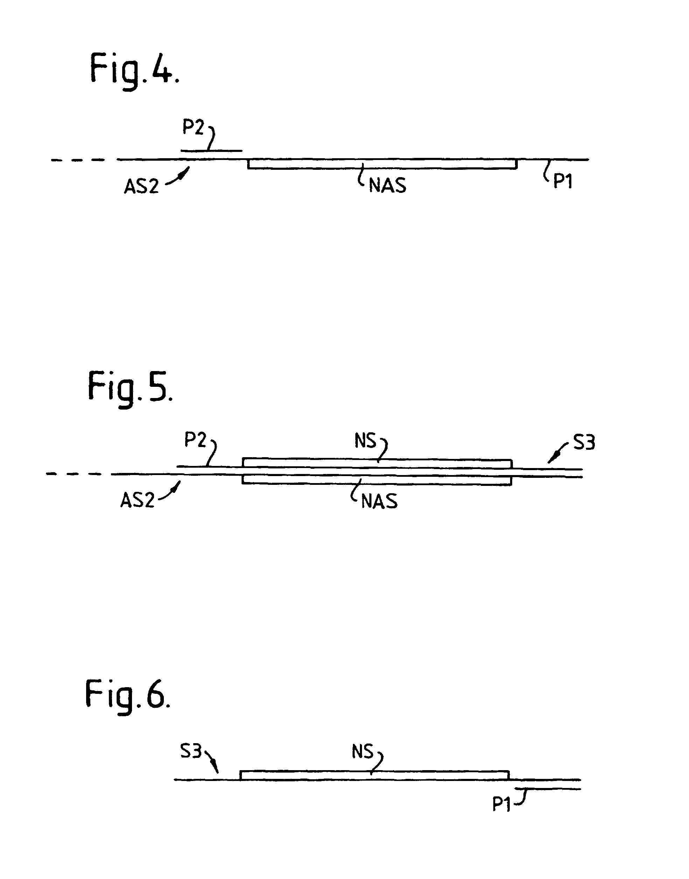

The steps of denaturing, annealing and DNA synthesis are then repeated. The other primer P2 which is able to anneal to the antisense strand AS2 at a position adjoining the other end of the desired gene, as shown in Fig. 4. In the subsequent step of synthesis a new DNA strand is formed which extends from primer P2 as far as an end complementary to primer P1, but then cannot extend further. This strand is designated S3 in Fig. 5.

The sequence of denaturing, annealing and DNA synthesis is continued repeatedly. During subsequent steps primers can anneal to any of the DNA strands which are present including strands such as S3 which contain only the desired sequence plus terminal portions corresponding to the primers. This is illustrated by Fig. 6. The consequence of this is that during subsequent repetitions of the cycle the production of DNA which contains only the desired nucleotide sequence plus the terminal portions corresponding to the primers far outstrips the production of all other DNA. After a number of repetitions the DNA present in the mixture is almost entirely of this type.

The steps of the reaction could be brought about in other ways : for instance reagents could be added to denature DNA into single strands and later removed to allow annealing to occur. Primers might be added only when required rather than initially. So also might the reagents for DNA synthesis. It is possible that the procedure could be started directly from RNA. The essence of the technique is repeatedly carrying out a cycle of steps comprising:

exposing a required nucleotide sequence in a nucleic acid strand,
annealing a primer oligonucleotide adjacent an end of the required sequence, and
synthesising a complementary nucleic acid strand extending from the primer,
these steps being carried out utilising a primer able to anneal to one nucleic acid strand adjacent to one end of the required sequence and a second primer able to anneal to the complementary nucleic acid strand adjacent the opposite end of the required sequence, thereby to produce clone strands of nucleic acid which are the required sequence with end portions determined by the two primers.

Subsequently such a clone sequence of nucleic acid is introduced into a non-human organism as extra genetic material. This transformed non-human organism is used to express the required polypeptide.

Usually the clone sequence will be produced as a double strand of nucleic acid, and is introduced into a non-human organism in this double strand form.

The polymerase chain reaction has been known for a number of years and is described in the literature. References are Saiki et al., Science 230 1350-1354 (1985), Scharf et al., Science 233 1076 (1986) and Saiki et al., Science 239 487 (1988).

The considerable advantage arising from use of the polymerase chain reaction is that it provides a very high proportion of DNA containing little more than the required nucleotide sequence. This makes it highly likely that when DNA is incorporated into an organism the transformed organism contains the required DNA rather than some other, unwanted DNA.

If a clone sequence is incorporated intact into an non-human organism which is used to express the polypeptide, the polypeptide will have terminal amino acid sequences coded by the PCR primers. These are referred to as 'motif' peptides and may or may not themselves contribute to the function of the polypeptide.

The primers which are used in the polymerase chain reaction must be capable of annealing to the original DNA at the required positions. However in order to do this it is not essential that they should be exactly complementary to the DNA at all positions, and they do not need to be unique to a single polypeptide coded by the nucleotide sequence between the primers.

Notably, it has been shown that in Fv antibody fragments both the heavy and light chains contain respective "framework regions" which have considerable homology from one antibody to another, especially within a species, together with three hypervariable regions also known as complimentarity determining regions (CDR). Amplification of antibody fragments by means of the polymerase chain reaction may be carried out using primers which correspond to portions of the framework regions flanking the hypervariable regions.

This was demonstrated by Orlandi, Winter et al in PNAS USA 86 3833 (1989) which discloses oligonucleotide primers able to anneal to regions at the ends of mouse nucleotide sequences coding for heavy chain variable domains and respectively kappa type light chain variable domains. As is there disclosed, these primers can be used for cloning light and heavy chain antibody fragments of mouse monoclonal antibodies independently of the binding specificity of the monoclonal antibodies concerned. These primers and some others are disclosed also in EP-A-368684 (Winter et al/Medical Research Council).

A characteristic of using this technique to clone the genes for a number of polypeptides, e.g. a series of antibody fragments or other proteins with homologous terminal regions, is that all the polypeptides produced will have N-terminal and C-terminal amino acid sequences (motif peptides) which may not correspond precisely to natural amino acid sequences but are coded by the primers used (and are therefore the same). Although these motif peptides are not necessarily identical to the natural amino acid sequence coded by the starting gene, they will at least resemble it closely and therefore are not truly "foreign".

It is possible that the motif peptide at a terminal portion of a polypeptide will differ to a limited extent from the amino acid sequence coded by the copy of a nucleotide primer at the end of the clone sequence of nucleic acid.

This can happen because of the way in which the clone sequence is introduced into an non-human organism. The primer sequence can incorporate a restriction site so that when the clone is incorporated into a vector such as a bacterial plasmid which is subsequently used to transform an organism, restriction enzyme can be used to cut away a terminal portion of the nucleic acid of the clone sequence. In this event, nucleotides from the vector will replace some or all of the nucleotides cut away from the clone sequence. These nucleotides from the vector are preferably prearranged to code for the same amino acids as the nucleotides from the clone which they replace, (or if not the same amino acids then a very similar sequence) in order that the motif peptide does not appear foreign but instead resembles a natural amino acid sequence. If the primer ends with an incomplete codon the vector should replace this with a complete codon.

Consequently, at least part of a motif peptide will be expressed from nucleotide sequence copied from a primer while part of a motif peptide may be expressed from nucleotide codons which are taken (wholly or partially) from elsewhere, but desirably do not substantially alter the amino acid sequence from that coded by the primer. In turn the primer has to resemble an existing nucleotide sequence in order to be able to anneal to a complementary sequence.

Broadly, the present invention arranges that a motif peptide will act as a target for a binding reagent. This reagent is then used in production of, or other work with, polypeptides which incorporate the motif peptide.

In a first aspect this invention provides a method of producing at least one polypeptide by culture of a genetically transformed organism comprising:

cloning a required portion of a nucleic acid sequence by means of the polymerase chain reaction utilising a pair of oligonucleotide primers thereby to obtain a clone sequence with terminal portions defined by the primers,
transforming an non-human organism with a said clone sequence resulting from the polymerase chain reaction or a copy therefrom,
culturing the transformed non-human organism to express the polypeptide coded by the clone sequence,

characterised by assaying or purifying the said polypeptide with a binding reagent displaying specific affinity towards an amino acid sequence in which there are at least three amino acids in sequence coded by a said primer.

A codon in the primer does of course code for both the identity of an amino acid and its position in a sequence.

A sequence of three amino acids is believed to be the minimum for specific affinity in binding and has been referred to as a "recognition kernel".

Usually, in this invention, the binding reagent will be intended to bind to a sequence of more than three amino acids but containing a sequence of at least three which are coded by the primer.

The requirement that the amino acid sequence contains a sequence of at least three amino acids coded by the primer allows for the possibility that the binding reagent is raised against an amino acid sequence which is slightly different from the sequence coded by the primer. Such a difference could be addition of one or more amino acids, especially when the primer has an incomplete terminal codon. It could be replacement of one or more amino acids by others.

If a degenerate primer is used to code the motif peptide, the binding reagents could be designed to have an affinity for a short region of at least three amino acids corresponding to a sequence of nucleotides which does not vary between the individual oligonucleotides in the degenerate primer.

Each primer used for the polymerase chain reaction may be a single oligonucleotide or, as mentioned earlier, may be a mixture of several oligonucleotides which are similar.

Preferably the binding reagent displays affinity towards an amino acid sequence including a sequence of four, five, six or more amino acids coded both as to identity and position by the primer.

It is likely that the binding reagent will display specific affinity towards an amino acid sequence of at least six amino acids, including the said sequence of at least three, preferably more, amino acids coded by the primer.

The overall sequence preferably contains at least six, possibly at least eight amino acids coded, both as to identity and position, by the primer. These amino acids may form a continuous sequence, or may be part of a sequence which includes some amino acids differing from whatever is coded by the primer. Preferably a majority of amino acids in a sequence to which the reagent displays affinity are coded by the primer. Possibly they will all be coded by the primer.

As explained above, a motif peptide may include amino acids expressed from nucleotide codons which did not originate from the primer. However, such nucleotides are desirably arranged not to give rise to any substantial difference in the expressed amino acid sequence, with the consequence that the resulting motif peptide will also be bound by the said binding reagent displaying affinity towards an amino acid sequence coded, at least partly, by the primer.

The binding reagent is preferably an antibody or an antibody fragment. Alternative binding reagents which could be used include antisense peptides, mimetic compounds resembling the binding site of an antibody, and affinity dyes.

Techniques for transforming a non-human organism with foreign nucleic acid have become well known and this invention is not limited to any type of non-human organism nor to any particular technique for effecting transformation. The nucleic acid sequence which is introduced may be a transcript from a clone made during the polymerase chain reaction rather than itself being a direct product of that reaction. It may be a complementary copy of inverse sense and, as mentioned above, a portion of the primer sequence may be removed before the remainder of the clone sequence is incorporated into the transformed organism.

As is well known, both strands of the DNA double helix represent coded information, but only one strand is utilised directly in protein synthesis. One of the two primers used in the polymerase chain reaction must be the primer for an antisense nucleic acid strand. It is therefore possible that an amino acid sequence to which there is specific affinity is a sequence expressed from part of a sense strand which is complementary to a primer rather than identical to a primer.

One advantage of this invention is that it avoids the introduction of a "foreign" amino acid sequence for use in assay or purification. This invention is especially valuable when applied to producing a plurality of different polypeptides with at least one primer being used for cloning all the respective nucleic acid strands. It then becomes possible to assay or purify the different polypeptides using similar antibody or antibody fragments with affinity for an amino acid sequence coded by the primer used in all the cloning steps. Thus, by utilising a binding reagent, e.g. antibody or fragment which binds to an amino acid sequence coded by the common primer, the same binding reagent can be utilised for more than one polypeptide. For each polypeptide this reagent can be sufficiently specific to bind the required polypeptide rather than anything else produced in the course of culturing the transformed organism but the reagent is not so specific as to be unique to a single polypeptide.

The invention is applicable to the production of polypeptides of a wide variety of types, so long as the original cloning of the nucleic acid is carried out by the polymerase chain reaction. However the invention is particularly applicable when the polypeptides come from a family of proteins with homologous terminal regions, so that primers which code for terminal regions can be used to clone a family of genes. An important example of such a family is immunoglobulin variable domains, when the primer can code for amino acid sequences resembling those in the framework region whereas the remainder of the polypeptide can include an antibody's hypervariable regions.

When the polypeptides are antibody variable domains and the primers code for amino acid sequences of the framework regions of those variable domains, it will be preferable to choose a C-terminal amino acid sequence to be recognised by binding reagent used in assay or purification. This C-terminal, unlike the N-terminal, is relatively exposed and remote from the binding site of the heavy chain variable domain. Consequently it is more accessible than the N-terminal, which will make it easier for the capture reagent to bind to the variable domain.

We have observed that for some Fv antibody fragments the accessibility of the C-terminal of the variable domain is somewhat affected by surrounding conditions, being enhanced by absorption onto a microtitre plate, or in the presence of a moderate electrolyte concentration such as 1M sodium chloride. These conditions appear to cause some modification to the conformation of the antibody fragment, without denaturing the protein.

The advantage of binding to the C-terminus of a polypeptide applies to both the heavy chain and light chain variable domains of antibodies. Moreover, a number of other proteins have a relatively exposed C-terminal portion so that the same advantages would be available.

When the polypeptides are antibody fragments it may be desirable to transform the same non-human organism to express both a desired fragment of the light or heavy chain and a corresponding fragment of the other chain. The two fragments can be allowed to associate together spontaneously, before carrying out the step of assay or purification, which step can utilise a motif peptide of only one of the two associated chains.

In a second aspect the invention provides the use of a kit of materials for carrying out the method according to the present invention, said kit comprising an oligonucleotide primer for use in the polymerase chain reaction and a binding reagent displaying specific binding affinity towards an amino acid sequence in which there are at least three amino acids in sequence coded by the said primer.

In another aspect this invention provides the use of an antibody, an antibody fragment or other binding reagent with specific binding affinity to one of certain amino acid sequences set out below, in the method according to the present invention.

When a reagent such as an antibody or antibody fragment binds to a polypeptide the binding is in accordance with an equilibrium reaction of the general form

$$A + P = AP$$

where A denotes the reagent, P denotes the polypeptide and AP denotes the complex of reagent bound to the polypeptide. The concentrations are related by an expression of the form

$$\frac{[AP]}{[A]\,[P]} = K_a$$

Where $K_a$ is a constant, referred to as the affinity constant. When there is specific binding affinity the value of $K_a$ will generally be $10^4$ or greater frequently substantially greater. For an antibody the value may be in the range $10^7$ to $10^9$. For an antisense peptide the value may be in the region of $10^5$.

Primers for use in the polymerase chain reaction typically contain between 15 and 45 nucleotides so that they code for an amino acid sequence of 5 to 15 amino acids.

An antibody with specific binding affinity to a motif peptide (amino acid sequence) can be obtained by a procedure which starts with chemical synthesis of the motif amino acid sequence itself or something similar to it. Methods for the chemical synthesis of peptides containing as many as 15 amino acids (and indeed somewhat more) are well known.

The amino acid sequence is then covalently conjugated to a carrier protein. Techniques for effecting such covalent conjugation are also well known. Next a suitable host animal is immunised with the conjugate in order to generate an immune response. After an appropriate delay, serum is collected from the host animal.

Polyclonal antibodies with specific binding affinity to the amino acid sequence can then be extracted by affinity chromatography using a chromatography column on which the amino acid sequence has been immobilized. The resulting polyclonal antibodies will generally be satisfactory for use in this invention where work is being carried out on a laboratory scale. However, if desired, it would be possible to use monoclonal antibody generated by standard techniques or an antibody fragment derived from these. This will generally be desirable if work is on a production scale because it is inconvenient to provide polyclonal antibodies in large quantities.

As a possible alternative to chemical synthesis of the motif peptide, if the polymerase chain reaction has already been used in the production of a polypeptide, that polypeptide might itself be used for raising antibodies which include antibodies binding to a motif amino acid sequence coded, at least partly, by an oligonucleotide primer. (Antibodies which bind specifically to the motif can be recovered on an affinity column with the motif sequence immobilised thereon). These antibodies could then be used when producing another polypeptide in accordance with the invention, using the same two primers or at least one of them.

List of Drawings

Figures 1 to 6, already referred to above, are diagrams illustrating the polymerase chain reaction.

Figures 7 to 9 and 11 to 13 are graphs illustrating results obtained by assays referred to in the Examples below.

Figure 10 illustrates the fusion of two nucleotide sequences.

Examples

The invention will be further explained and illustrated by the following Examples which relate to the production of antibody fragments with variable regions cloned using the polymerase chain reaction as referred to above.

In these Examples materials are denoted by abbreviations as follows:

PBS = Phosphate Buffered Saline ($0.01M\ NaH_2PO_4/Na_2HPO_4$, yielding pH 7, together with 0.15M NaCl

PBSA = PBS + 0.1% by weight sodium azide

PBST = PBS + 0.15% by weight Tween 20

PPD = purified protein derivative, supplied by Statens Seruminstitut, Copenhagen

BCIP/NBT = 5-bromo-4-chloro-3-indoyl phosphate and p-nitroblue tetrazolium chloride which is a substrate system for alkaline phosphatase and is supplied by Promega, Southampton

PNPP =      para-nitrophenyl phosphate, which is an alternative substrate system for alkaline phosphatase, available from Sigma

Tween20 =      a nonionic surfactant available from Sigma

Primers for amplifying heavy chain gene

The primer VH1 FOR 2 is disclosed in EP-A-368684. It has the sequence designated SEQ ID No 1 in the sequence listing at the end of this description.

A related primer which has two additional nucleotides at the 3' end is disclosed in EP-A-368684 as VH1 FOR. This is also disclosed in Orlandi, Winter at al PNAS USA 86 3383 (1989).

VH1 FOR 2 is referred to as a forward primer and is itself antisense. It was designed to anneal with the 3' end of the sense strand of sequences coding for mouse heavy chain variable domains. It codes (indirectly) for a C-terminal amino acid sequence (i.e. peptide) which is included, with its C-terminal on the right, in SEQ ID No 2 in the sequence listing and repeated separately as SEQ ID No 3. A sense strand, complementary to this primer codes directly for the same amino acid sequence. This sense strand is also included in SEQ 1D No 2 and incorporates a Bst EII recognition site, as indicated in bold type with the cleavage site arrowed.

The primer VH1 BACK is disclosed in EP-A-368684 and in the Orlandi et al paper referred to above. It is in fact a mixture of very similar primers differing at certain nucleotides. It has the sequence designated SEQ ID No 4 in the sequence listing at the end of this description, where S is used to denote C or G, M to denote A or C, R to denote A or G and W to denote A or T. It is a sense strand and was designed to anneal with the 3' end of the antisense strand of any nucleotide sequences coding for mouse heavy chain variable domains. This primer incorporates as a Pst I recognition site whose position is indicated and has an incomplete codon at the 5' end. If this codon is completed appropriately the sequence codes for either of two N-terminal amino acid sequences shown as SEQ ID No 5 in which the alternative acids Glu and Gln are shown as Glx.

Primers for amplifying light chain gene

A suitable forward primer has the sequence designated SEQ ID No 7 in the sequence listing at the end of this description.

This primer is itself antisense and is designed to anneal with the 3' end of the sense strand of sequences coding for mouse kappa-type light chain variable domains.

The complementary sense strand is shown as SEQ ID No 8. It incorporates a Xho I recognition site as indicated.

A suitable back primer is VK1 BACK as shown in EP-A-368684.

Example 1

Raising of Antibodies

An amino acid sequence designated Mot1 and given in the sequence listing below as SEQ ID No 4 was synthesised chemically. As can be seen it consists of the sequence SEQ ID No 2 coded by the VH1 FOR 2 primer together with two additional lysines to enhance its solubility and facilitate eventual immobilization on an affinity chromatography column. This peptide, designated Mot1 was covalently conjugated onto the carrier protein PPD and the resulting conjugate, designated Mot1-PPD was used to inoculate rabbits.

Antibodies with specific binding affinity towards the motif peptide Mot1 were isolated by affinity purification as follows: 5mg of the peptide MotI was immobilised onto 3mls wet volume of CNBr-activated Sepharose 4B (Pharmacia) according to that manufacturers instructions. The resulting material was packed into a liquid chromatography column.

Serum (5ml) from rabbits immunised as above was loaded onto the column which was then washed through with PBSA to remove unbound material (the fallthrough) after which the antibodies bound to the Mot1 peptide on the column were eluted with 4M aqueous $MgCl_2$, then dialysed overnight into PBSA.

Analysis of the original serum and the affinity purified fraction by polyacrylamide gel electrophoresis showed that the procedure had isolated pure immunoglobulin.

Solutions of antibodies were tested for ability to bind to the Mot1 peptide by means of an ELISA assay, as follows.

A microtitre plate was incubated with a solution of Mot1-PPD conjugate (0.1µg/litre in carbonate buffer) at 37°C overnight, to allow the conjugate to become immobilised on the plate. Next the plate was washed and wells of the plate were exposed to test solution (containing the polyclonal antibodies derived as above) for 1 hour to allow binding to the immobilized peptide. The plate was then washed again and exposed to a solution of goat anti-rabbit antibodies conjugated to alkaline phosphatase (available from Sigma). These bind to the antibodies (which are of course rabbit antibodies) which have bound to the Mot1. Finally after a further washing the plate is exposed to PNPP substrate solution for alkaline phosphatase, which is converted by that enzyme to a coloured product. The optical density at 405nm is read after a period of about 30 minutes and is a measure of binding to the Mot1-PPD conjugate.

To check the specificity of the binding a similar assay was carried out in which the initial solution of Mot1-PPD conjugate was replaced with a solution of PPD itself so that this assay gives a measure of binding to PPD.

If there is more binding to Mot1-PPD conjugate than to PPD itself in these assays, they show that there is binding to the Mot1 peptide.

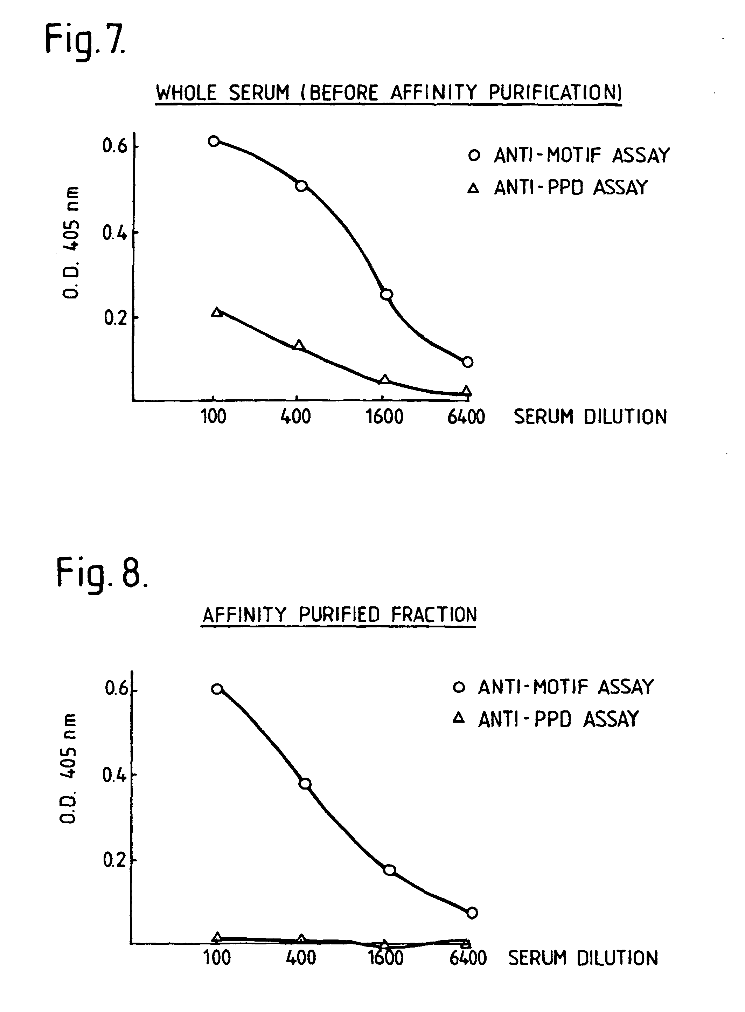

Serum from immunised rabbits was diluted to various extents and tested in these assays. The results are shown graphically in Figure 7 of the drawings in which the results of binding to Mot1-PPD are shown with circles and the results of binding to PPD alone are shown with triangles.

This shows that the serum contained antibodies which bind specifically to the Mot1 peptide as well as antibodies to PPD protein.

The affinity purified fraction was diluted and tested similarly. The results are shown graphically in Figure 8 of the drawings.

This shows that the affinity purification has successfully isolated antibodies which bind to the Mot1 peptide.

Example 2

Anti-lysozyme Fragments

In Example 5 of EP-A-368684, Winter et al describe the production of Fv antibody fragments from a known monoclonal anti-lysozyme antibody designated "D1.3".

The affinity purified antibodies from Example 1 were used in a capture ELISA assay of Fv anti-lysozyme fragments of this type. These fragments had the amino acid sequence set out as SEQ ID No 2 at the C-terminal end of the heavy chain.

Microtitre plates were incubated with 1μg/ml lysozyme incarbonate buffer overnight at 37°C so that lysozyme is immobilised in the plate wells.

Next the plates were washed with PBST and wells of the plate were exposed for 1 hour at room temperature of about 20°C to a test solution containing the Fv fragments diluted in PBST, for these to bind to the lysozyme.

The plate was then washed again and exposed to a solution of the affinity purified antibodies, for these to bind to the motif sequence at the C-terminal of the heavy chain of the fragments.

After this the plate was washed again, and exposed to a solution of goat anti-rabbit antibodies conjugated to alkaline phosphatase (Sigma) followed by washing and exposure to PNPP substrate solution for the alkaline phosphatase.

The optical density at 405nm after about 30 minutes is a measure of binding to the plate. The binding is dependent on the presence of the Fv fragments and the optical density should therefore provide a measure of the concentration of these in the test solution.

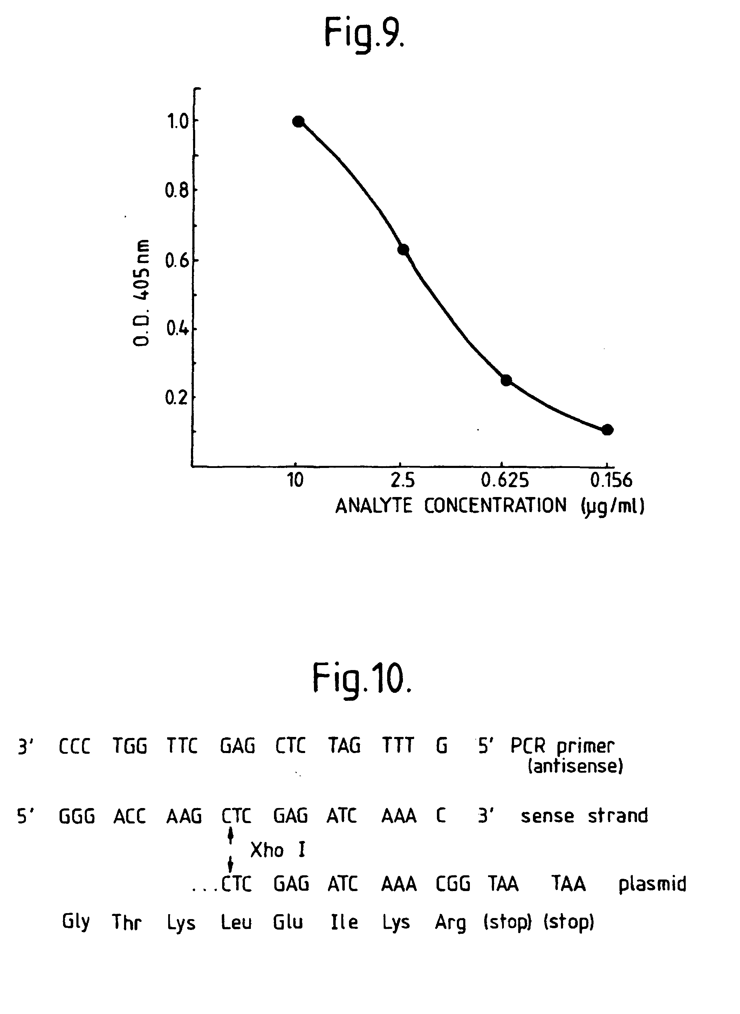

It was found that this was indeed the case. A plot of optical density against concentration of Fv fragments in the test solution is shown as Figure 9. The results fit a sigmoid curve, as can be seen.

This demonstrates that antibodies raised against the twelve amino acid sequence Mot 1 had specific binding affinity to polypeptides incorporating the ten amino acid motif peptide.

The affinity purified antibodies from Example 1 were also used in a Western blot procedure for the detection of the Fv antilysozyme fragments, as follows.

A test sample containing the fragments was first subjected to polyacrylamide gel electrophoresis. The resulting gel was electroblotted onto a 0.2μm nitrocellulose membrane. This membrane was incubated successively with

(i) solution of the affinity purified antibodies (2μg/ml in PBSA + 0.05% Tween20),

(ii) solution of goat anti-rabbit alkaline phosphatase conjugate (Sigma: diluted 1 in 2000 in PBSA + 0.05% Tween20),

(iii) substrate for the alkaline phosphatase leading to development of colour where the alkaline phosphatase conjugate has been bound to the membrane.

This technique was demonstrated on a test sample which was supernatant from an E. coli culture expressing the Fv fragments. One lane of the electrophoresis was stained with silver stain and showed the presence of numerous bands corresponding to various E. coli proteins. A second lane was used for the Western blot procedure described above and this showed a single band of colour at the molecular weight appropriate for $V_H$ fragments. (In the Fv fragments the $V_H$ and $V_L$ chains are not covalently linked and they separate during this analytical procedure). Hence the anti-motif antibodies were selectively detecting the Fv fragments in the presence of various other proteins.

Example 3

Anti-hormone Fragments

Fv antibody fragments were derived from mouse monoclonal antibodies with binding affinity to a human hormone (hCG), by procedures similar to those described by Winter et al in EP-A-368684.

The nucleotide sequence coding for the heavy chain variable region was amplified by means of the PCR reaction using the primers VH1 FOR 2 and VH1 BACK already referred to above. The resulting cloned DNA sequence had end portions corresponding to the primers. It was introduced into a plasmid, in the course of which procedure the end portions of the clone strands of DNA were cut away with restriction enzyme at the Bst EII and Pst I sites. The plasmid provided nucleotide sequence which exactly replaced that between the Bst EII recognition site and the terminus of the clone strands (the 3' terminus of the sense strand, corresponding to the C-terminus of the expressed polypeptide). Stop codons were added by the plasmid. The plasmid also provided nucleotide sequence which replaced that between the Pst I recognition site and the other terminus of the clone strands of DNA (while eliminating some of the redundancy in the VH1 BACK primer and completing the incomplete codon at the 5' end of that primer).

In the same way the nucleotide sequence coding for the light chain variable region was amplified by means of the PCR reaction using the forward primer shown as SEQ ID No 8 and the VK1 BACK primer shown in EP-A-368684. The resulting cloned DNA sequence had end portions corresponding to these primers.

The cloned DNA sequence was also introduced into the plasmid referred to above. In the course of this the end portion from the Xho I recognition site was cut away by restriction enzyme and replaced with nucleotide sequence from the plasmid. This completed the codon at the 3' end of the sense strand and also added stop codons, as shown diagrammatically in Fig. 10. The nucleotide sequence from the plasmid is also shown as SEQ ID No 9. The motif peptide coded by the sequence after introduction into the plasmid is shown as SEQ ID No 10.

The constructed plasmid containing both cloned sequences of DNA was used to transform E.coli which then expressed light and heavy chains. These spontaneously associated with each other as Fv fragments with the motif peptide set out as SEQ ID No 3 at the C-terminus of the heavy chain and the motif set out as SEQ ID No 10 at the C-terminus of the light chain.

Samples taken from E. coli cultures expressing these fragments were subjected to cell lysing conditions and then tested by the Western blot procedure of the previous Example. A single band of colour was observed at the molecular weight for a $V_H$ fragment, denoting the presence of the desired Fv fragments.

This demonstrates that the anti-motif antibodies can be used in assaying a plurality of different polypeptides where these are derived via a cloning step using the same primer in the polymerase chain reaction.

The peptide shown as SEQ ID No 10 could be synthesised and used to raise antibodies, as in Example 1. These antibodies could be used, as in Examples 2 and 3 to assay for expressed Fv fragments or for light chain vairable domains.

Example 4

Affinity purified antibodies from Example 1 were used in a direct ELISA assay of three Fv fragments. These were the Fv anti-lysozyme fragments of Example 2, the Fv anti-hCG fragments of Example 3, and an Fv antibody fragment with binding agent affinity to glucose oxidase which was made by procedures similar to those set out in Example 3.

For each Fv fragment, microtitre plates were sensitised by incubating test solutions which were dilutions of Fv (made up in sensitisation buffer) for 1 hour at 37°C. Plates were then incubated for 1 hour at room temperature with a 1ug/ml solution of affinity purified rabbit anti-motif antibody made up in PBST. The plates were then incubated for 1 hour at room temperature with a 1/1000 dilution of goat anti-rabbit antibodies conjugated to alkaline phosphatase (Sigma). Finally, plates were developed with pNPP substrate (Sigma). Plates were washed thoroughly with PBST between each incubation.

As in Examples 2 and 3, the optical density at 405 nm is a measure of binding to the plate, and hence a measure of the concentration of the Fv antibodies in the test solution.

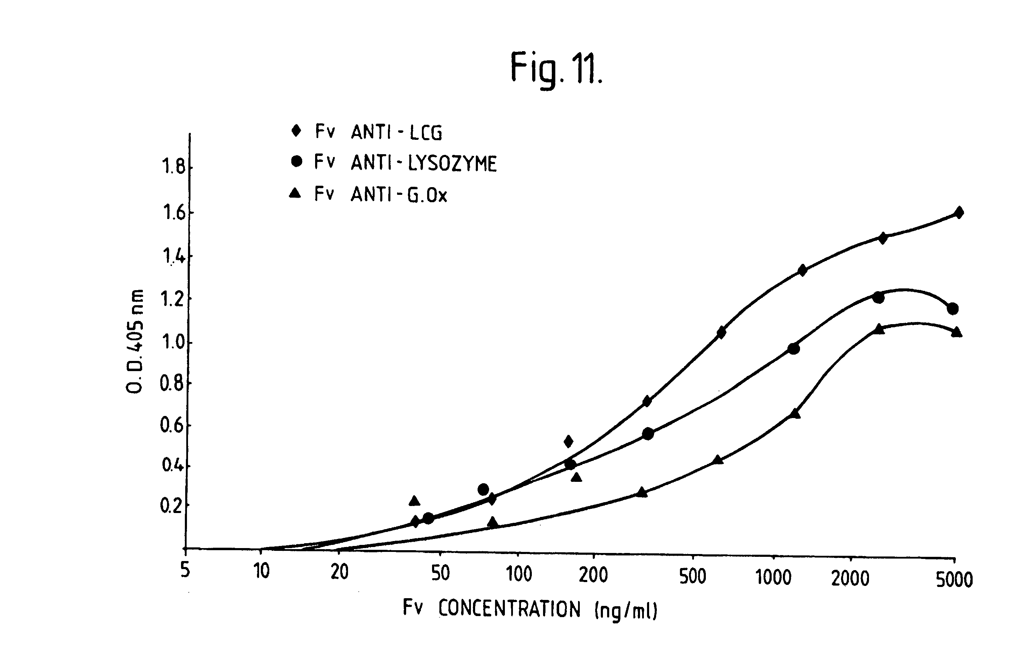

A plot of optical density against concentration is shown as Figure 11. All three antibodies were detectable by this assay at concentrations of 50 nanograms/ml and above.

Example 5

Rabbit antibodies produced as in Example 1 were used to monitor the production of Fv anti-hCG fragments in a fermenter. These fragments were expressed by transformed E.coli as in Example 3. The E.coli was in 5 litre fermenters. The growth medium was:

| | |
|---|---|
| $Na_2HPO_4$ | 12.0 g/litre |
| $KH_2PO_4$ | 6.0 g/litre |
| NaCl | 0.5 g/litre |
| $NH_4Cl$ | 5.0 g/litre |
| L-proline | 60 mg/litre |
| glycerol | 30 g/litre |
| $MgSO_4.6H_2O$ | 0.49 g/litre |
| $CaCl_2.2H_2O$ | 15 mg/litre |
| Thiamine hydrochloride | 1 mg/litre |
| Yeast extract (Beta Labs) | 10 g/litre |

The growth temperature was 25°C or 30°C and the pH was controlled at 6.8 by auto addition of 40% (w/v) NaOH. The impeller speed was 500rpm and aeration rate was 0.1 v/v/m. The inoculum (1% v/v) was grown overnight on M9P medium supplemented with yeast extract (5g/liter) in a shaking incubator at 25°C. During the first 15 hours of fermentation, the impeller speed and air flow rate were gradually increased to maxima of 650 rpm and 0.3 v/v/m respectively.

Fv anti-hCG fragment production was induced by the addition of filter sterilised isopropyl-B-D-thiogalactopyranoside, IPTG (0.5M; 1ml/liter) during late exponential growth phase (12-15 hours from inoculation). Samples (5mls) were removed at known intervals for analysis and centrifuged for 15 minutes @ 3,000 rpm. The supernatent was then filtered through a 0.2 μm microfilter. Cells were lysed by a combination of osmotic shock and enzymatic treatment and the lysates passed through a 0.2um microfilter. The resulting supernatent and lysate fractions were assayed using anti-motif antibodies in a direct ELISA assay, as described in the previous example.

The assay was standardised with a set of standards - diluted from a stock solution of Fv anti-hCG - which had been purified to homogeneity on hCG-Sepharose and determined spectrophotometrically (using an extinction coefficient of $A_{280}$ = 1.9 as calculated from sequence information).

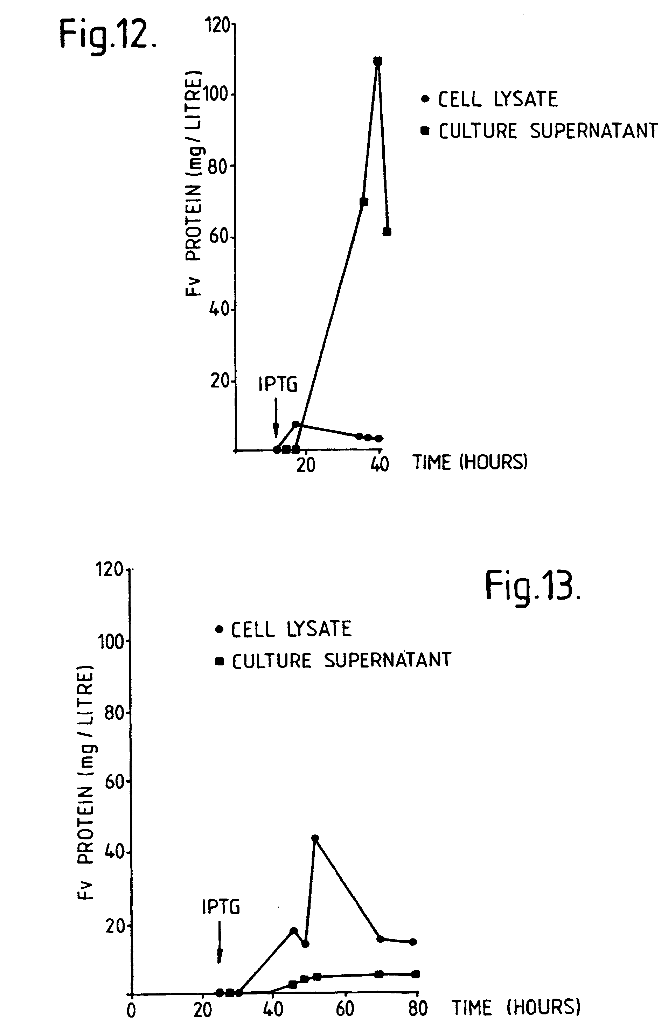

The results of these assays for fermentations at 25°C and 30°C are shown as Figures 12 and 13 respectively. Each figure is a plot of protein concentration against time. Protein in the culture supernatant is shown by squares, protein in the cell lysate is shown by circles.

As shown by Figure 12 compared with Figure 13, Fv anti-hCG was produced much more efficiently at 25°C (terminal yield was 62mg/litre) than at 30°C (terminal yield in was 15mg/litre). Furthermore, at the lower temperature, the Fv was found almost exclusively (90%+) in the supernatant (which this makes recovery of Fv easier). In contrast, at the higher temperature most of the Fv was found to be in the lysate fraction.

Example 6

This example demonstrates the use of antibody to a motif peptide to recover Fv fragments incorporating the motif peptide from a feedstock which resembled a fermentor broth.

Rabbit anti-motif antibody was prepared and isolated by affinity chromatography as described in Example 1. This procedure was repeated until a stock of 6mgs of motif-specific antibody had been built up. This preparation was concentrated to 1mg/ml in an ultrafiltration cell (Amicon), fitted with a membrane with molecular-weight cut-off of 8,000 Daltons (Amicon YM8). The concentrated antibody preparation was mixed with an equal volume of a buffer solution chosen so that the mixture contained 0.1M $NaHCO_3$, 0.5M NaCl and was at pH 8.3 and coupled onto 2 grams of chemically-activated agarose (CNBr Sepharose 4B, Pharmacia) according to that manufacturer's instructions. Unreacted CNBr groups were blocked by washing with an excess of 0.1M tris, pH 8 according to Pharmacia's instructions.

The resulting motif-specific immunoadsorbent, prepared as above, was packed in a glass column (Pharmacia) and fitted to a standard liquid chromatography set-up (Pharmacia). 100mls of a feedstock comprising 6 μg/ml Fv anti-lysozyme and 1mg/ml bovine albumin (Sigma) was loaded onto the column. This feedstock was representative of a fermentor broth. Unbound protein was removed by washing with PBS. Bound Fv was eluted by washing with aqueous 4M $MgCl_2$ and dialysed into PBS.

Example 7

A monoclonal anti-lysozyme antibody incorporated in its variable domain an amino acid sequence which was the same as that shown in SEQ ID No 2, except that valine at position 6 was replaced by leucine.

This anti-lysozyme antibody was bound to a microtitre plate. This was then exposed to a solution of the polyclonal antibody of Example 1. Binding of this polyclonal antibody to the anti-lysozyme antibody was demonstrated by the ELISA assay described in Example 1.

This demonstrates that the specific binding affinity was present despite one change in the amino acid sequence.

A further experiment confirmed that the antibody was binding to the relevant amino acid sequence rather than to some other site. Lysozyme was bound to a microtitre plate. Then the monoclonal anti-lysozyme antibody was allowed to bind to the lysozyme. The antibody of Example 1 did not bind to these anti-lysozyme complexes, because the whole variable domain of the antibody was blocked by the lysozyme.

SEQUENCE LISTING

    (1) GENERAL INFORMATION:

        (i) APPLICANT:

            (A) NAME: Unilever NV
            (B) STREET: Weena 455
            (C) CITY: Rotterdam
            (E) COUNTRY: The Netherlands
            (F) POSTAL CODE (ZIP): 3013 AL

            (A) NAME: Unilever plc
            (B) STREET: Unilever House, Blackfriars
            (C) CITY: London
            (E) COUNTRY: England
            (F) POSTAL CODE (ZIP): EC4P 4BQ

        (ii) TITLE OF INVENTION: Polypeptide production.

        (iii) NUMBER OF SEQUENCES: 10

        (iv) COMPUTER READABLE FORM:

            (A) MEDIUM TYPE: Floppy disk
            (B) COMPUTER: IBM PC compatible
            (C) OPERATING SYSTEM: PC-DOS/MS-DOS
            (D) SOFTWARE: PatentIn Release #1.0, Version #1.25 (EPO)

        (vi) PRIOR APPLICATION DATA:

            (A) APPLICATION NUMBER: GB 9216983.8
            (B) FILING DATE: 11-AUG-1992

    (2) INFORMATION FOR SEQ ID NO: 1:

        (i) SEQUENCE CHARACTERISTICS:

            (A) LENGTH: 32 base pairs
            (B) TYPE: nucleic acid
            (C) STRANDEDNESS: single
            (D) TOPOLOGY: linear

(ii) MOLECULE TYPE: DNA primer

(iii) ANTI-SENSE: YES

(xi) SEQUENCE DESCRIPTION: SEQ ID NO: 1:

```
TGA GGA GAC GGT GAC CGT GGT CCC TTG GCC CC                    32
```

(2) INFORMATION FOR SEQ ID NO: 2:

(i) SEQUENCE CHARACTERISTICS:

(A) LENGTH: 32 base pairs

(B) TYPE: nucleic acid

(C) STRANDEDNESS: single

(D) TOPOLOGY: linear

(iii) ANTI-SENSE: NO

(ix) FEATURE:

(A) NAME/KEY: CDS

(B) LOCATION: 3..32

(ix) FEATURE:

(A) NAME/KEY: misc_feature

(B) LOCATION: 17..23

(D) OTHER INFORMATION: /note= "Bst EII recognition site"

(ix) FEATURE:

(A) NAME/KEY: misc_feature

(B) LOCATION: 17..18

(D) OTHER INFORMATION: /note= "Bst EII cleavage site"

(xi) SEQUENCE DESCRIPTION: SEQ ID NO: 2:

```
GG GGC CAA GGG ACC ACG GTC ACC GTC TCC TCA                   32
   Gly Gln Gly Thr Thr Val Thr Val Ser Ser
     1               5               10
```

(2) INFORMATION FOR SEQ ID NO: 3:

(i) SEQUENCE CHARACTERISTICS:

(A) LENGTH: 10 amino acids

(B) TYPE: amino acid

(D) TOPOLOGY: linear

(ii) MOLECULE TYPE: peptide

(xi) SEQUENCE DESCRIPTION: SEQ ID NO: 3:

```
Gly Gln Gly Thr Thr Val Thr Val Ser Ser
 1               5                   10
```

(2) INFORMATION FOR SEQ ID NO: 4:

    (i) SEQUENCE CHARACTERISTICS:

        (A) LENGTH: 22 base pairs
        (B) TYPE: nucleic acid
        (C) STRANDEDNESS: single
        (D) TOPOLOGY: linear

    (ii) MOLECULE TYPE: DNA primer

    (iii) ANTI-SENSE: NO

    (ix) FEATURE:

        (A) NAME/KEY: misc feature
        (B) LOCATION: 9..14
        (D) OTHER INFORMATION: /note= "Pst I recognition site"

    (ix) FEATURE:

        (A) NAME/KEY: misc_feature
        (B) LOCATION: 13..14
        (D) OTHER INFORMATION: /note= "Pst I cleavage site"

    (xi) SEQUENCE DESCRIPTION: SEQ ID NO: 4:

```
AG GTS MAR CTG CAG SAG TCW GG                                    22
```

(2) INFORMATION FOR SEQ ID NO: 5:

    (i) SEQUENCE CHARACTERISTICS:

        (A) LENGTH: 8 amino acids
        (B) TYPE: amino acid
        (C) STRANDEDNESS: single
        (D) TOPOLOGY: linear

    (ii) MOLECULE TYPE: peptide

    (v) FRAGMENT TYPE: N-terminal

    (xi) SEQUENCE DESCRIPTION: SEQ ID NO: 5:

```
Gln Val Gln Leu Gln Glx Ser Gly
 1               5
```

(2) INFORMATION FOR SEQ ID NO: 6:

    (i) SEQUENCE CHARACTERISTICS:

      (A) LENGTH: 12 amino acids
      (B) TYPE: amino acid
      (C) STRANDEDNESS: single
      (D) TOPOLOGY: linear

  (ii) MOLECULE TYPE: peptide

  (v) FRAGMENT TYPE: C-terminal

  (xi) SEQUENCE DESCRIPTION: SEQ ID NO: 6:

```
    Lys Lys Gly Gln Gly Thr Thr Val Thr Val Ser Ser
    1               5                   10
```

(2) INFORMATION FOR SEQ ID NO: 7:

  (i) SEQUENCE CHARACTERISTICS:

      (A) LENGTH: 22 base pairs
      (B) TYPE: nucleic acid
      (C) STRANDEDNESS: single
      (D) TOPOLOGY: linear

  (ii) MOLECULE TYPE: DNA primer

  (iii) ANTI-SENSE: YES

  (xi) SEQUENCE DESCRIPTION: SEQ ID NO: 7:

```
    G TTT GAT CTC GAG CTT GGT CCC                                    22
```

(2) INFORMATION FOR SEQ ID NO: 8:

  (i) SEQUENCE CHARACTERISTICS:

      (A) LENGTH: 22 base pairs
      (B) TYPE: nucleic acid
      (C) STRANDEDNESS: single
      (D) TOPOLOGY: linear

  (iii) ANTI-SENSE: NO

  (ix) FEATURE:

      (A) NAME/KEY: misc feature
      (B) LOCATION: 10..15
      (D) OTHER INFORMATION: /note= "Xho I recognition site"

  (ix) FEATURE:

      (A) NAME/KEY; misc_feature
      (B) LOCATION: 10..11
      (D) OTHER INFORMATION: /note= "Xho I cleavage site"

  (xi) SEQUENCE DESCRIPTION: SEQ ID NO: 8:

```
GGG ACC AAG CTC GAG ATC AAA C                                          22
```

(2) INFORMATION FOR SEQ ID NO: 9:

    (i) SEQUENCE CHARACTERISTICS:

        (A) LENGTH: 21 base pairs
        (B) TYPE: nucleic acid
        (C) STRANDEDNESS: single
        (D) TOPOLOGY: linear

    (iii) ANTI-SENSE: NO

    (ix) FEATURE:

        (A) NAME/KEY: misc_feature
        (B) LOCATION: 1..2
        (D) OTHER INFORMATION: /note= "Xho I cleavage site"

    (xi) SEQUENCE DESCRIPTION: SEQ ID NO: 9:

```
CTC GAG ATC AAA CGG TAA TAA                                            21
```

(2) INFORMATION FOR SEQ ID NO: 10:

    (i) SEQUENCE CHARACTERISTICS:

        (A) LENGTH: 8 amino acids
        (B) TYPE: amino acid
        (C) STRANDEDNESS: single
        (D) TOPOLOGY: linear

    (ii) MOLECULE TYPE: peptide

    (v) FRAGMENT TYPE: C-terminal

    (xi) SEQUENCE DESCRIPTION: SEQ ID NO: 10:

```
Gly Thr Lys Leu Glu Ile Lys Arg
1                   5
```

## Claims

1. A method of producing at least one polypeptide by culture of a genetically transformed non-human organism comprising

   cloning a required portion of a nucleic acid sequence by means of the polymerase chain reaction utilising a pair of oligonucleotide primers thereby to obtain a clone sequence with terminal portions defined by the primers, transforming a non-human organism with a said clone sequence resulting from the polymerase chain reaction or a copy therefrom, culturing the transformed non-human organism to express the polypeptide coded by the clone sequence,

**characterised by** assaying or purifying the said polypeptide with a binding reagent displaying specific affinity towards an amino acid sequence in which there are at least three amino acids in sequence coded by a said primer.

2.  A method according to claim 1 applied to the production of a plurality of separate polypeptides, the method comprising

     a said step of cloning a respective portion of nucleic acid for each polypeptide, utilising a pair of oligonucleotide primers and the polymerase chain reaction, at least one of the primers being used for all of the said polypeptides,
     a said step of transforming a non-human organism, for each polypeptide,
     a said step of culturing the respective organism to express each polypeptide, and
     assaying or purifying each polypeptide, using the same said binding reagent, which reagent displays affinity towards an amino acid sequence in which there are at least three amino acids in sequence coded by the primer used for all the polypeptides.

3.  A method according to claim 1 or claim 2 wherein said amino acid sequence contains at least six amino acids.

4.  A method according to claim 1 or claim 2 wherein said amino acid sequence contains at least six amino acids coded by the primer.

5.  A method according to any one of the preceding claims wherein a primer used in the step of cloning is a mixture of oligonucleotides.

6.  A method according claim 2 wherein each polypeptide is an antibody variable domain.

7.  A method according to claim 2 wherein each polypeptide is an antibody variable domain, and the said binding reagent displays affinity towards an amino acid sequence at the C-terminal thereof.

8.  A method according to claim 7 wherein each polypeptide is an antibody heavy chain variable domain.

9.  A method according to claim 7 wherein each polypeptide is an antibody light chain variable domain.

10. A method according to any one of the preceding claims wherein

     the said nucleic acid sequence codes for a fragment of one chain of an antibody,
     the non-human organism is transformed with a nucleic acid sequence coding for a second chain of the antibody fragment as well as with the said nucleotide sequence,
     culturing the non-human organism expresses both chains of the antibody fragment,
     and the two chains associate together before the said step of assaying or purifying.

11. A method according to any one of the preceding claims wherein the said binding reagent is an antibody or antibody fragment.

12. A method according to any one of the preceding claims wherein the said binding reagent has specific affinity to the amino acid sequence given herein in SEQ ID No 3 or the sequence given herein in SEQ ID No 10.

13. Use of a kit in the polymerase chain reaction according to the method of claims 1 -12 comprising an a) an oligonucleotide primer; b) a reagent having specific binding affinity to an amino acid sequence on which there are at least 3 amino acids in sequence coded by the said primer.

14. Use of a kit according to claim 13 wherein the primer corresponds to a nucleotide sequence coding for a framework region of an antibody variable domain.

15. Use of a kit according to claim 14 wherein the primer is as shown in SEQ ID No. 1 or SEQ ID No. 7.

16. Use of a binding reagent according to the method of claims 1 to 12 having specific binding affinity to the amino acid sequence given herein in SEQ ID No. 3 or the sequence given herein in SEQ ID No. 10.

**Patentansprüche**

1. Verfahren zur Herstellung von mindestens einem Polypeptid durch Kultivierung eines genetisch transformierten nicht-menschlichen Organismus, umfassend

   das Klonieren des erforderlichen Teils einer Nukleinsäuresequenz durch die Polymerase-Kettenreaktion unter Einsatz eines Paars aus Oligonukleotidprimern, um so eine Klonsequenz zu erhalten, deren terminale Teile durch die Primer definiert sind,
   Transformieren eines nicht-menschlichen Organismus mit der Klonsequenz, die aus der Polymerase-Kattenreaktion resultiert oder einer Kopie davon,
   Kultivieren des transformierten, nicht-menschlichen Organismus zur Expression des durch die Klonsequenz kodierten Polypeptids,

   **dadurch gekennzeichnet, daß** das Polypeptid einem Assay unterzogen wird oder aufgereinigt wird, mit einem Bindungsreagenz, das eine spezifische Affinität für eine Aminosäuresequenz zeigt, in der mindestens drei Aminosäuren in Sequenz durch einen der Primer kodiert werden.

2. Verfahren gemäß Anspruch 1, angewandt auf die Herstellung einer Vielzahl von getrennten Polypeptiden, wobei das Verfahren umfaßt

   einen Schritt zum Klonieren des jeweiligen Teils einer Nukleinsäure für jedes Polypeptid, unter Verwendung eines Paares von Oligonukleotidprimern und der Polymerase-Kettenreaktion, wobei mindestens einer der Primer für alle Polypeptide verwendet wird,
   einen Schritt zur Transformation eines nicht-menschlichen Organismus für jedes Polypeptid,
   einen Schritt zur Kultivierung des jeweiligen Organismus zur Expression jedes Polypeptids, und

   wobei jedes Polypeptid einem Assay unterzogen wird oder aufgereinigt wird, unter Verwendung des gleichen Bindungsreagenzes, wobei das Bindungsreagenz Affinität für eine Aminosäuresequenz zeigt, in der mindestens drei Aminosäuren in Sequenz durch den Primer kodiert werden, der für alle Polypeptide verwendet wird.

3. Verfahren gemäß Anspruch 1 oder Anspruch 2, wobei die Aminosäuresequenz mindestens sechs Aminosäuren enthält.

4. Verfahren gemäß Anspruch 1 oder Anspruch 2, wobei die Aminosäuresequenz mindestens sechs Aminosäuren enthält, die von dem Primer kodiert werden.

5. Verfahren gemäß mindestens einem der vorhergehenden Ansprüche, wobei der in dem Schritt der Klonierung verwendete Primer eine Mischung aus Oligonukleotiden ist.

6. Verfahren gemäß Anspruch 2, wobei jedes Polypeptid eine variable Domäne eines Antikörpers ist.

7. Verfahren gemäß Anspruch 2, wobei jedes Polypeptid eine variable Domäne eines Antikörpers ist und das Bindungsreagenz eine Affinität für eine Aminosäuresequenz an dessen C-terminaten Ende zeigt.

8. Verfahren gemäß Anspruch 7, wobei jedes Polypeptid eine variable Domäne einer schweren Kette eines Antikörpers ist

9. Verfahren gemäß Anspruch 7, wobei jedes Polypeptid eine variable Domäne einer leichten Kette eines Antikörpers ist.

10. Verfahren gemäß mindestens einem der vorhergehenden Ansprüche, wobei

    die Nukleinsäuresequenz für ein Fragment von einer Kette eines Antikörpers kodiert,
    der nicht-menschliche Organismus mit einer Nukleinsäuresequenz transformiert wird, die für eine zweite Kette des Antikörperfragments kodiert, sowie mit der Nukleotidsequenz,

    wobei die Kultivierung des nicht-menschlichen Organismus zur Expression beider Ketten des Antikörperfragments führt,

und wobei die beiden Ketten vor dem Schritt des Assays oder der Aufreinigung miteinander assoziieren.

11. Verfahren gemäß mindestens einem der vorhergehenden Ansprüche, wobei das Bindungsreagenz ein Antikörper oder ein Antikörperfragment ist.

12. Verfahren gemäß mindestens einem der vorhergehenden Ansprüche, wobei das Bindungsreagenz eine spezifische Affinität für die Aminosäuresequenz aufweist, die hierin in SEQ ID Nr. 3 angegeben ist oder die Sequenz, die hierin in SEQ ID Nr. 10 angegeben ist.

13. Verwendung eines Kits bei der Polymerase-Kettenreaktion gemäß dem Verfahren nach den Ansprüchen 1 bis 12, umfassend a) einen Oligonukleotidprimer; b) ein Reagenz mit spezifischer Bindungsaffinität für eine Aminosäuresequenz, in der mindestens drei Aminosäuren in Sequenz vorkommen, die durch den Primer kodiert werden.

14. Verwendung des Kits gemäß Anspruch 13, wobei der Primer zu einer Nukleotidsequenz korrespondiert, die für eine Gerüstregion einer variablen Domäne eines Antikörpers kodiert.

15. Verwendung des Kits gemäß Anspruch 14, wobei der Primer wie in SEQ ID Nr. 1 oder SEQ ID Nr. 7 gezeigt ist.

16. Verwendung eines Bindungsreagenzes gemäß dem Verfahren nach den Ansprüchen 1 bis 12, das eine spezifische Bindungsaffinität an die Aminosäuresequenz aufweist, die hierin in SEQ ID Nr. 3 angegeben ist oder an die Sequenz, die hierin in SEQ ID Nr. 10 angegeben ist.


## Revendications

1. Méthode de production d'au moins un polypeptide par culture d'un organisme non humain génétiquement transformé comprenant le fait de

cloner une portion requise d'une séquence d'acide nucléique au moyen de la réaction en chaîne de polymérase employant une paire d'amorces oligonucléotidiques pour obtenir ainsi une séquence de clone avec des portions terminales définies par les amorces,
transformer un organisme non humain avec ladite séquence de clone obtenue par la réaction en chaîne de polymérase ou l'une de ses copies,
cultiver l'organisme non humain transformé pour exprimer le polypeptide codé par la séquence de clone,

**caractérisée par** le test ou la purification dudit polypeptide avec un réactif de liaison présentant une activité spécifique vis-à-vis d'une séquence d'acides aminés dans laquelle il y a au moins trois acides aminés dans la séquence codée par une desdites amorces.

2. Méthode selon la revendication 1 appliquée à la production d'une pluralité de polypeptides séparés, la méthode comprenant

une dite étape de clonage d'une portion respective d'acide nucléique pour chaque polypeptide, employant une paire d'amorces oligonucléotidiques et la réaction en chaîne de polymérase, au moins une des amorces étant utilisée pour tous lesdits polypeptides,
une dite étape de transformation d'un organisme non humain pour chaque polypeptide
une dite étape de culture de l'organisme respectif pour exprimer chaque polypeptide, et
le test ou la purification de chaque polypeptide; en utilisant le même dit réactif de liaison, ce réactif présentant une activité vis-à-vis d'une séquence d'acides aminés dans laquelle il y a au moins trois acides aminés dans la séquence codée par l'amorce utilisée pour tous les polypeptides.

3. Méthode selon la revendication 1 ou la revendication 2, dans laquelle ladite séquence d'acides aminés contient au moins six acides aminés.

4. Méthode selon la revendication 1 ou la revendication 2, dans laquelle ladite séquence d'acides aminés contient au moins six acides aminés codés par l'amorce.

5. Méthode selon l'une quelconque des revendications précédentes, dans laquelle une amorce utilisée dans l'étape de clonage est un mélange d'oligonucléotides.

6. Méthode selon la revendication 2, dans laquelle chaque polypeptide est un domaine variable d'anticorps.

7. Méthode selon la revendication 2, dans laquelle chaque polypeptide est un domaine variable d'anticorps, et ledit réactif de liaison présente une affinité vis-à-vis d'une séquence d'acides aminés à son extrémité C-terminale.

8. Méthode selon la revendication 7, dans laquelle chaque polypeptide est un domaine variable de chaîne lourde d'anticorps.

9. Méthode selon la revendication 7, dans laquelle chaque polypeptide est un domaine variable de chaîne légère d'anticorps.

10. Méthode selon l'une quelconque des revendications précédentes, dans laquelle

ladite séquence d'acide nucléique code un fragment d'une chaîne d'un anticorps
l'organisme non humain est transformé avec une séquence d'acide nucléique codant une seconde chaîne du fragment d'anticorps ainsi qu'avec ladite séquence nucléotidique,
la culture de l'organisme non humain exprime les deux chaînes du fragment d'anticorps,
et les deux chaînes s'associent l'une avec l'autre avant ladite étape de test ou de purification.

11. Méthode selon l'une quelconque des revendications précédentes, dans laquelle ledit réactif de liaison est un anticorps ou un fragment d'anticorps.

12. Méthode selon l'une quelconque des revendications précédentes, dans laquelle ledit réactif de liaison a une affinité spécifique pour la séquence d'acides aminés donnée ici dans SEQ ID No 3 ou la séquence donnée ici dans SEQ ID No 10.

13. Utilisation d'un kit dans la réaction en chaîne de polymérase selon la méthode des revendications 1-12 comprenant a) une amorce oligonucléotidique ; b) un réactif ayant une affinité de liaison spécifique pour une séquence d'acides aminés dans laquelle il y a au moins trois acides aminés dans la séquence codée par ladite amorce.

14. Utilisation d'un kit selon la revendication 13, dans laquelle l'amorce correspond à une séquence d'acide nucléique codant une région de structure d'un domaine variable d'anticorps.

15. Utilisation d'un kit selon la revendication 14, dans laquelle l'amorce est présentée dans SEQ ID No. 1 ou SEQ ID No. 7.

16. Utilisation d'un réactif de liaison selon la méthode de l'une quelconque des revendications 1 à 12, ayant une affinité de liaison spécifique pour la séquence d'acides aminés donnée ici dans SEQ ID No. 3 ou la séquence donnée ici dans SEQ ID No. 10.

Fig.1.

NS

S1

AS1

NAS

Fig.2.

NS

S1

P1

Fig.3.

NS

S1

AS2

NAS

P1

Fig.4.

Fig.5.

Fig.6.

# Fig. 7.

WHOLE SERUM (BEFORE AFFINITY PURIFICATION)

o ANTI-MOTIF ASSAY
△ ANTI-PPD ASSAY

O.D. 405 nm

SERUM DILUTION

# Fig. 8.

AFFINITY PURIFIED FRACTION

o ANTI-MOTIF ASSAY
△ ANTI-PPD ASSAY

O.D. 405 nm

SERUM DILUTION

# Fig.9.

# Fig.10.

```
3'  CCC  TGG  TTC  GAG  CTC  TAG  TTT  G   5'  PCR primer
                                                    (antisense)

5'  GGG  ACC  AAG  CTC  GAG  ATC  AAA  C   3'   sense strand
                     ↑  Xho I
                     ↓
               ...CTC  GAG  ATC  AAA  CGG  TAA   TAA    plasmid

    Gly  Thr  Lys  Leu  Glu  Ile  Lys  Arg  (stop) (stop)
```

# Fig. 11.

◆  Fv  ANTI - LCG
●  Fv  ANTI - LYSOZYME
▲  Fv  ANTI - G.Ox

Fv CONCENTRATION (ng/ml)

O.D. 405 nm

Fig.12.

- CELL LYSATE
- CULTURE SUPERNATANT

Fig.13.

- CELL LYSATE
- CULTURE SUPERNATANT